# EUROPEAN PATENT APPLICATION

(11) **EP 0 722 694 A2**
(43) Date of publication of application: **24.07.1996**
(21) Application number: 96300433.8
(22) Date of filing: 23.01.1996
(51) Int. Cl.: A61B 17/06

(54) **A package for surgical sutures provided with a needle**

(30) Priority: 24.01.1995 BR 9500286
(71) Applicant: ETHICON, INC., Somerville, NJ 08873 (US)
(72) Inventor: Bordignon, Marcos A., Centro, Sao José dos Campos, Sao Paulo (BR); Januzelli, José L.L., Sao Dimas, Sao Jose dos Campos Sao Paulo (BR)
(74) Representative: Fisher, Adrian John

(57) **Abstract**

A package for a surgical suture, provided with a needle, is described which comprises a first elongated retaining panel (2), provided with a needle-securing means (1) and openings (12) for receiving auxiliary pins for winding sutures (S); at least one second retaining panel (3, 4, 5) adjacent said first retaining panel (2) and joined to a longitudinal edge thereof by means of a fold line (14); a first auxiliary retaining panel (6) joined to a transverse edge of the first retaining panel (2) by a fold line (15); a second auxiliary retaining panel (7), joined to a transverse edge of the first retaining panel (2) opposite the transverse edge which joins the first auxiliary retaining panel (6) to the first retaining panel (2); and a third auxiliary retaining panel (8), joined along a fold line (17) to the longitudinal edge of the first retaining panel (2) opposite the one adjacent said at least one retaining panel (3, 4, 5), with the third retaining panel (8) having means for providing engagement and removal of a needle and a corresponding suture from said needle-securing means (1) of the first retaining panel (2), without the need for opening the package completely; characterized in that said at least one second retaining panel (3, 4, 5) comprises a flap (A) secured to said retaining panel (3, 4, 5) by means of a folding line (18) and having a free end (21), when said panel (3, 4, 5) is folded along said folding line (14) over the first retaining panel (2), rests against the edge of said needle-securing means (1), defining a ramp; and said means for providing engagement and removal of the needle and suture, provided in the third retaining panel (8), comprises a window (13) provided in the third retaining panel (8), which superimposes, at least partially, said needle-securing means (1) of the first retaining panel (2), when the third retaining panel (8) is folded over the first retaining panel (2) along the fold line (17).

## Description

### Technical field

The present invention relates to a folding package or retainer for surgical products, more specifically to a package made up of a plurality of folding panels particularly suitable for retaining a sterilized surgical suture made of a polymeric material provided with an elastic memory and having at least one needle secured thereto.

### Background art

Various types of packages for surgical sutures are already known from the state of the art, the packages varying in construction depending upon the kind of suture and purpose. In general, the main fuction of these packages is to protect the suture and the respective needle(s) during transportation, handling and storage, which take place until it reaches the final user. Moreover, the improved packages of the state of the art have low manufacturing costs and are constructed to be easily opened, allowing prompt access to the suture-needle assembly and easy removal from the package for use.

The most pertinent prior art with respect to the present invention is that represented by Brazilian patent applications PI 9301757 and PI 9004553, in the name of Johnson & Johnson, the contents of which are incorporated herein by reference.

The former patent application, namely PI 9301757, is the most relevant to the present invention and refers to a folding package for surgical products, particularly adapted for holding a filamentary surgical product, comprising:
- first and second elongated retaining panels, which are foldably joined to each other along their longitudinal edges, the filamentary surgical product being wound in a non-crossed manner on the first retaining panel;
- a first auxiliary retaining panel, foldably joined to a transverse edge of the first retaining panel; and
a first elongated securing panel, whose longitudinal edge is foldably joined to the longitudinal edge of the first retaining panel opposite the second retaining panel, the first locking panel having a substantially transverse weakened tear line.

The package of that application of the prior art further comprises:
- a tongue-and-slot type securing device, the respective slot being disposed in a transversely intermediate position of the first retaining panel; and
- openings provided in said first retaining panel, distributed along substantially semicircular paths, through which pins can pass to facilitate the winding of the filamentary surgical product in a non-crossed manner.

The teachings of Brazilian patent application PI 9301757 provide a quite satisfactory solution to the problems which previously existed, these being due to the "elastic memory" of the suture thread, which, after having been taken out of the package in which it was wound in a "8-shaped" manner, tended to have an undulating shape, making it difficult to use, principally when the thread was made of a multifilamentary or monofilamentary synthetic material. However, some shortcomings to the device of PI 9301757 still remain, namely:
(a) the need for tearing or folding a portion of a side panel of the package in order to expose the needle remains as a complication to the process of removing the suture, using surgical pincers when in the operating theatre; and
(b) in some cases, the possibility that the suture gets entangled, in the foam or other material used for securing the needle, as it is removed from the package.

### Object of the invention

It is, therefore, an object of the present invention to provide a package, for a surgical suture and needle, which overcomes the above problems which are still encountered in the prior art.

### Summary of the invention

According to the present invention a package for surgical sutures provided with a needle comprises:
- a first elongated retaining panel, provided with a needle-securing means and openings for receiving auxiliary pins for winding sutures;
- at least one second retaining panel adjacent the first retaining panel and joined to a longitudinal edge thereof by means of a fold line;
- a first auxiliary retaining panel joined to a transverse edge of the first retaining panel by a fold line;
- a second auxiliary retaining panel joined to a transverse edge of the first retaining panel, opposite the transverse edge which joins the first auxiliary retaining panel to the first retaining panel; and
- a third auxiliary retaining panel, joined along a fold line to the longitudinal edge of the first retaining panel opposite the edge adjacent the second retaining panel, with the third retaining panel having means for providing engagement and removal of the needle and its corresponding suture from the needle-securing means of the first retaining panel, without the need for opening the package completely;
wherein the means for providing engagement and removel of the needle and the suture, provided in the third retaining panel, comprises a window which at least partially superimposes the needle-securing means of the first retaining panel, when the third retaining panel is folded over the first retaining panel along the folding line.

According to a preferred embodiment of the invention, the second retaining panel comprises a flap, secured thereto by means of a fold line, having a free end which, when the second retaining panel is folded along its fold line over the first retaining panel, rests on the edge of said needle-securing means, thereby defining a ramp which facilitates the removal of the suture thread from the package through the window.

According to a further preferred embodiment of the invention, a flap is provided, adjacent a transverse edge of the window, capable of being hinged along a fold line with a free edge thereof facing inwardly of the window, the flap being capable of cooperating with that provided on the second retaining panel, to prevent the suture from becoming entangled in the needle-securing means, when the needle is taken out of the package through the window.

The package according to the present invention has two main advantages over those of the prior art:
firstly that the needle is more easily taken out of the package by virtue of the window, through which one can have access to the needle. In this way there is no need for the package to be opened in order for the needle to be visible and/or accessible; and
secondly that the suture is prevented from becoming entangled in the needle-securing means, when the needle is taken out through the window, thanks to the flap provided on the second retaining panel which allows the thread to slide over the needle-securing means. This effect is further improved when another flap is provided below the window, which, when the window is closed, cooperates with the first-mentioned flap, thereby providing more room for the thread to slide out of the package through the window.

### Brief description of the drawings

The present invention will now be described in greater detail with reference to the accompanying drawings, in which:
figure 1 shows a plan view of a package for a surgical suture provided with a needle, as taught by the closest prior art;
figure 2 shows a plan view of a second embodiment of the package of the closest prior art;
figure 3 shows a plan view of a preferred embodiment of the present invention.

### Detailed description of the invention

Firstly a description will be given of the closest prior art with respect to the present invention.

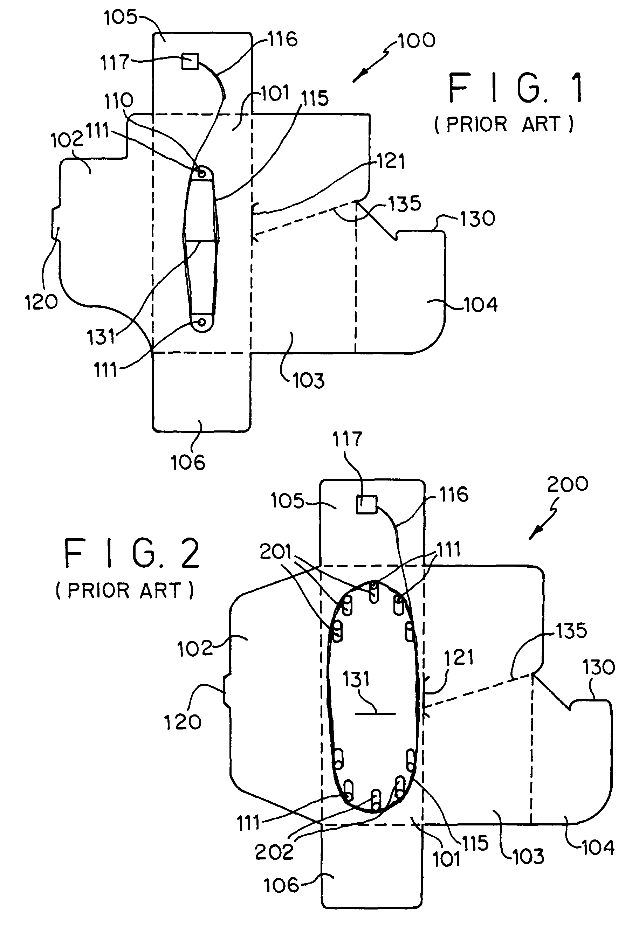

As can be seen from figure 1, a package according to a first embodiment of the prior art is generically designated by the number 100.

Package 100 comprises a first rectangular retaining panel 101, foldably connected along respective longitudinal edges to a second retaining panel 102 and to a first securing panel 103, both also rectangular. Panel 103 is also foldably connected, along its longitudinal edge, to a second securing panel having a reduced longitudinal dimension when compared with the corresponding dimensions of panels 101, 102 and 103.

Package 100 further includes first and second auxiliary retaining panels 105 and 106, which are approximately square and foldably joined to the shorter transverse edges of the first retaining panel 101.

In one embodiment of the package of the prior art, two apertures 110 are provided in the first retaining panel 101, through which respective pins 111 are introduced, the function of which is to facilitate the manual winding of a suture 115 having a needle 116 secured to one of its ends. Optionally, the suture 115 may have a needle 116 secured to each of its ends, or even no needle at all. Also, the package 100 may contain more than one suture 115.

Suture 115 is wound in a non-crossed manner around the pins 111, so that the turns of the suture 115 can assume a substantially oval configuration as they expand when released.

Upon folding the panels 101 - 106 to form the package 100, needle 116 is advantageously held in a compartment separated from suture 115, thus preventing accidental cutting of suture 115 and also preventing damage occuring to the cutting edges of needle 116.

Package 100 further includes a first securing device, made up of a first tongue 120, provided at the free longitudinal edge of second retaining panel 102, and by a first slot 121, capable of receiving first tongue 120 and disposed at the edge joining first retaining panel 101 to first securing panel 103. The function of first securing device 120,121 is to hold second retaining panel 102 folded over first retaining panel 101, with suture 115 and second auxiliary retaining panel 106 between them.

In an optional embodiment of package 100 of the prior art, second auxiliary retaining panel 106 can be folded over second retaining panel 102, which in turn is folded over first retaining panel 101 and the suture 115.

A second securing device is also provided, which consists of a second tongue 130, located at the free edge of second securing panel 104 and whose axis of symmetry is parallel to the line joining retaining panel and securing panel 101-104 and to the longitudinal axis of the package 100 when closed.

Second securing device further comprises a second slot 131, disposed substantially in the centre of first retaining panel 101 between apertures 110, and transversely to the longitudinal axis of first panel 101.

Second securing device 130,131 serves to immobilize second locking panel 104 on the rear face of first retaining panel 101, holding package 100 in its folded configuration. In this way, the existence of second locking panel 130,131 means that first locking device 120,121 is merely optional.

As shown in figure 1, first securing panel 103 has a weakened line 135 which can be torn so that a portion of first securing panel 103 and subsequently first auxiliary retaining panel 105 can be raised by a user in order to obtain access to the suture 115 and to the needle 116.

Figure 2 relates to an embodiment of the closest prior art with respect to the present invention, according to which a package 200 has several portions identical to those which make up package 100 shown in figure 1. Accordingly, these portions will be designated in figure 2 with the same reference numbers of figure 1.

As can be seen, the main difference between package 200 and the one designated by reference number 100 in figure 1 simply relates to the number and disposition of apertures 201,102 in first retaining panel 101.

In order for the package 200 to hold surgical sutures provided with any degree of elastic memory, without the risk of a portion thereof being unduly removed by second securing device 130,131, the turns of suture 115 should be wound along a substantially circular or oval path. This is because, in sutures made from materials having lower degrees of elastic memory, as for instance those made of a material of animal or vegetable origin ("cat gut", silk, cotton, etc.) or a polymeric woven multifilamentary material (nylon, polyester, polyethylene, polypropilene, etc.), the tendency to expand centrifugally after winding can be insufficient to guarantee that the turns of suture 115 displace as far as the edges of the first retaining panel 101, and thereby leave the central portion of panel 101, where second slot 131 is disposed, totally free.

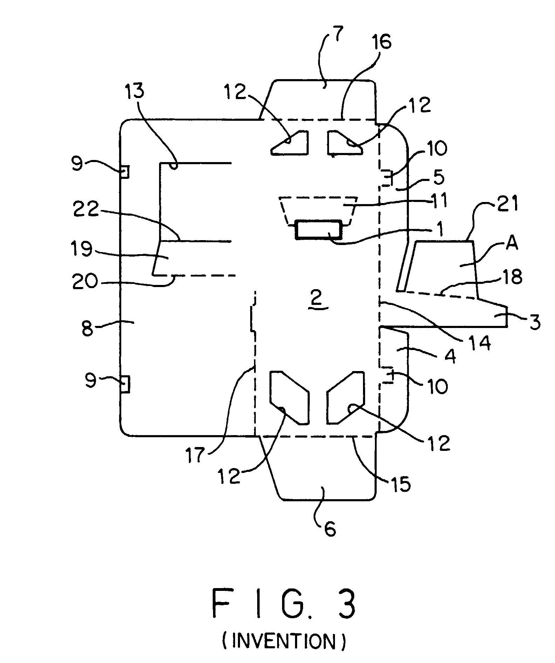

Thus, according to the embodiment of the prior art shown in figure 2, apertures 201, 202 are provided along substantially semicircular paths in respective first and second groups adjacent the shorter transverse edges of first retaining panel 101. In this way, during the step of winding suture 115, a plurality of pins 111 pass through apertures 201,202 and facilitate the arrangement of the turns of the suture 115 long the oval path which is already adjacent the edges of first retaining panel 101, independently of a centrifugal expansion of the turns of suture 115. Turning now to the present invention, with particular reference to figure 3, a package is shown, according to a preferred embodiment of the invention, which comprises a first elongated retaining panel 2 provided, approximately in its central region, with a securing means, for a needle (not shown) attached to the end of a suture (not shown), in the form of a foam pad 1.

A second retaining panel, formed by three adjacent panel sections 3, 4 and 5, is joined to a longitudinal edge of first retaining panel 2 by means of a fold line 14. A central section 3 of second retaining panel 3, 4, 5 is provided with a flap A, joined to it along a fold line 18. The function of the flap A will be described in greater detail below.

First retaining panel 2 has, further, adjacent to its two opposite retaining edges, first and second auxiliary retaining panels 6 and 7, joined respectively to first retaining panel 2 and along fold lines 15 and 16. In the conventional way, openings 12 are provided close to the first retaining panel 2, to receive pins (not shown) intended to facilitate the winding of the suture (not shown) in an "O"-configuration along the edges of first retaining panel 2.

A third retaining panel 8 is also provided, which is joined to first retaining panel 2 by a fold line 17 along the longitudinal edge of first panel 2 opposite the edge adjacent second retaining panel 3, 4, 5. Third retaining panel 8 is also provided with a window 13, the purpose of which will be described in greater detail later. Third retaining panel 8 also has closing means 9 cabable of cooperating with corresponding closing means 10 located on first retaining panel 2, to close the package, as will be explained later.

The package in accordance with the invention is assembled as folows:
- foam pad 1 is secured to first retaining panel 2, after which the needle (not shown) is secured thereto;
- central section 3 of second retaining panel 3, 4, 5 is folded over first retaining panel 2, so as to cover part of foam pad 1 and part of the needle (not shown), which is secured thereto;
- the suture (not shown), provided with the needle, is wound in the conventional manner, around winding pins (not shown) which are received in openings 12. Since this step of winding the suture around pins is common to the prior art, it will not be described here in greater detail;
- sections 4 and 5 of second retaining panel 3, 4, 5 are simultaneously folded over first retaining panel 2;
- first an second auxiliary retaining panels 6 and 7 are also simultaneously folded over first retaining panel 2, with panels 6 and 7 also partly covering sections 4 and 5 of second retaining panel 3, 4, 5; and
- finally, third retaining panel 8 is folded over the whole assembly, effecting the inter-engagement of the cooperating closing means 9 and 10.

In this way, the needle (not shown) remains visible through window 13 and can be held and withdrawn therethrough, without the need to even partially open the package.

It should be noted that window 13 has a flap 19, adjacent a transversal edge, having a free end 21, which flap 19, intended to facilitate the withdrawal of the suture accompanied by the needle, cooperates with flap A provided on central section 3 of second retaining panel 2, to prevent the suture from getting entangled or caught in foam pad 1 as it is pulled from the package. In this way, while flap A of second retaining panel 3, 4, 5 forms a "ramp" for the suture to slide over without entangling in foam pad 1, flap 19 allows, by raising its free end 21, a larger clearance for the suture to pass through window 13 when it leaves the "ramp", so as to ensure that it does not become entangled.

The provision of flap A on said second retaining panel 3, 4, 5 is, however, optional and may be eliminated, in the case when pad 1, of foam or any other material, has a substantially trapezoidal form and is not parallelipipedal, as shown.

It should be readily appreciated that the folding package for surgical products described above merely represents a preferred embodiment, and as such the scope of the present invention should be limited only by the terms and interpretation of the following claims.

## Claims

1. A package for surgical sutures provided with a needle, which comprises:
- a first elongated retaining panel (2), provided with needle-securing means (1) and openings (12) for receiving auxiliary pins for winding sutures (S);
- at least one second retaining panel (3, 4, 5) adjacent said first retaining panel (2) and joined to a longitudinal edge thereof by means of a fold line (14);
- a first auxiliary retaining panel (6) joined to a transverse edge of said first retaining panel (2) by a fold line (15);
- a second auxiliary retaining panel (7), joined to a transverse edge of said first retaining Panel (2) opposite the transverse edge joining said first auxiliary retaining panel (6) to said first retaining panel (2); and
- a third auxiliary retaining panel (8), joined along a fold line (17) to the longitudinal edge of said first retaining panel (2), opposite that adjacent said at least one second retaining panel (3, 4, 5), with said third retaining panel (8) having means for providing engagement and removal of a needle and a corresponding suture from said needle-securing means (1) of said first retaining panel (2), without the need for opening the package completely;
characterized in that said means for providing engagement and removal of the needle and suture, comprises a window (13), provided in said third retaining panel (8), which at least partially superimposes said needle-securing means (1) of said first retaining panel (2), when said third retaining panel (8) is folded over said first retaining panel (2), along said fold line (17).

2. A package according to claim 1, characterized in that said at least one second retaining panel (3, 4, 5) comprises a flap (A), joined thereto by means of a fold line (18), said flap (A) having a free end (21), which, when said panel (3, 4, 5) is folded over said first retaining panel (2), along said fold line (14), rests against an edge of said needle-securing means (1), defining a ramp over which the suture can slide when it is taken out of the package through said window (13).

3. A package according to claim 2, characterized by being provided with a flap (19), adjacent a transverse edge of said window (13), capable of being hinged along a fold line (20) with a free edge (22) thereof facing inwardly of said window (13), said flap (19) being capable of cooperating with said flap (A), provided on said at least one second retaining panel (3, 4, 5), to prevent the suture from becoming entangled in said needle-securing means (1), when the needle is taken out of the package through said window (13).
